# EUROPÄISCHE PATENTSCHRIFT

(11) **EP 1 363 701 B1**
(45) Veröffentlichungstag und Bekanntmachung des Hinweises auf die Patenterteilung: **11.11.2009**
(21) Anmeldenummer: 02735107.1
(22) Anmeldetag: 28.02.2002
(51) Int. Cl.: A61N 2/02

(54) **BEHANDLUNGSVORRICHTUNG MIT BEHANDLUNGSLIEGE UND BEHANDLUNGSGERÄT**
TREATMENT DEVICE WITH TREATMENT BED AND TREATMENT APPARATUS
DISPOSITIF DE TRAITEMENT AVEC LIT DE TRAITEMENT ET APPAREIL DE TRAITEMENT

(30) Priorität: 01.03.2001 DE 10109849
(43) Veröffentlichungstag der Anmeldung: 26.11.2003
(73) Patentinhaber: Bio-Magnetic Therapy Systems GmbH, 81371 München (DE)
(72) Erfinder: MARKOLL, Richard, 81925 München (DE)
(74) Vertreter: Zimmermann, Gerd Heinrich
(86) Internationale Anmeldenummer: PCT/EP2002/002184
(87) Internationale Veröffentlichungsnummer: WO 2002/074033

(56) Entgegenhaltungen:
- EP-A- 0 661 079
- EP-A- 0 809 993
- WO-A2-97/00639
- DE-C- 955 895
- GB-A- 2 341 099
- US-A- 3 830 233
- US-A- 4 167 182
- US-A- 5 088 475

## Beschreibung

### Gebiet der Erfindung

Die vorliegende Erfindung betrifft eine Behandlungsvorrichtung mit einer Behandlungsliege und einem Behandlungsgerät, die zur Behandlung von Patienten im Kopfbereich geeignet ist.

Eine Behandlung von Patienten mit beispielsweise Tinnitusbeschwerden ist erfolgreich durch Bestrahlungen möglich, beispielsweise durch Bestrahlung mit bestimmten Signalen, wie pulsierenden Feldern, die über ein Magnetfeld aufgebracht werden. Dazu wird der Patient und insbesondere dessen die Beschwerden aufweisender Kopfbereich, über eine verhältnismäßig lange Dauer, etwa in der Größenordnung einer Stunde, einer Bestrahlungsbehandlung ausgesetzt. Dies bedeutet, dass der Patient über eine vorbestimmte Zeit auf einer Behandlungseinrichtung sitzt oder liegt, wobei die Signale, beispielsweise die bestimmten Signale, wie pulsierende Felder, die über ein Magnetfeld aufgebracht werden, auf seinen Kopf in geeigneter Weise aufgebracht werden.

Damit die Strahlen in geeigneter Weise und wirkungsvoll auf den Kopfbereich des Patienten aufgebracht werden können, muss eine Behandlungsliege in Verbindung mit dem Behandlungsgerät ferner sicherstellen, dass der Patient seine Behandlungsposition während der Behandlung nicht drastisch verändert.

### Stand der Technik

Behandlungsliegen mit verschiedenen Verstellmechanismen für die verschiedensten Behandlungen sind in der Technik bekannt. Beispielsweise sind Massageliegen bekannt, die im Bereich, in dem der Kopf des Patienten ruht, eine kopfförmige Aussparung aufweisen, so dass ein auf dem Bauch liegender Patient ohne seinen Kopf zu verdrehen und somit Verspannungen zu erzeugen, atmen und bequem liegen kann, solange die Massagebehandlung an ihm durchgeführt wird.

Auch für andere Arten von Behandlungen bzw. Untersuchungen sind entsprechend geformte Liegen bekannt.

Zur Behandlung im Kopfbereich sind ebenfalls verschiedene Behandlungsdrähte bekannt. Beispielsweise sind padförmige Auflagen, kopfhörerartige Behandlungsgeräte oder Hauben zur Bestrahlungstherapie, auch mit Magnetfeldern, bekannt. Auch röhrenförmige Elemente, die einen festgelegten Durchmesser der Aufnahmeöffnung für den Kopf des Patienten haben, werden eingesetzt.

In der EP 0 661 079 A1 wird ein Magnettherapiegerät mit einer Behandlungsliege beschrieben. Die dort beschriebene Behandlungsliege umfasst mitunter eine Vielzahl von Magnetfelderzeugungseinheiten, die parallel auf einer Befestigungsbasis angeordnet sind und im Kopfbereich eines Patienten zur Anwendung kommen. Hierzu offenbart das Dokument ein Kopfauflageteil, auf dem der Kopf des Patienten positioniert werden kann.

Die WO 97/00639 offenbart ein elektromagnetisches Therapiegerät, bei dem ein magnetischer Flussgenerator im Rückenteil eines Behandlungssitzes beweglich angeordnet ist und über Schienen auf unterschiedliche Höhen bewegt werden kann.

### Darstellung der Erfindung

Der Erfindung liegt die Aufgabe zugrunde, eine Behandlungsvorrichtung mit Behandlungsliege und Behandlungsgerät vorzuschlagen, die bei einer längeren Behandlung eines Patienten im Kopfbereich, insbesondere durch Bestrahlung, eine bequeme, definierte Lage des Patienten auf der Liege und bezüglich des Behandlungsgerätes ermöglichen, wobei gleichzeitig die Behandlungsliege bzw. das Behandlungsgerät und Behandlungsvorrichtung auf verschiedene Körpermaße eines Patienten anpassbar bleiben.

Diese Aufgabe wird durch eine Behandlungsvorrichtung mit den Merkmalen des Anspruchs 1 gelöst.

Im Hinblick auf die Behandlungsliege liegt der Erfindung der Gedanke zugrunde, dass die Behandlungsliege durch ihre langgestreckte Aussparung, die sich in Längsrichtung der Behandlungsliege erstreckt, geeignet ist, ein Behandlungsgerät aufzunehmen, das durch eine geeignete Vorrichtung, die innerhalb der langgestreckten Aussparung verschiebbar ist, auf die Körperlänge eines Patienten justiert werden kann, so dass das Behandlungsgerät geeigneterweise im Kopfbereich des Patienten ansetzt. Dabei ist die langgestreckte Aussparung, von der auch mehrere vorgesehen sein können, wenn das Behandlungsgerät über mehrere Träger fixiert werden soll, so angeordnet, dass sie sich vom Kopfende der Behandlungsliege aus so weit erstreckt, dass sowohl Patienten, die durch ihre Körpergröße die gesamte Behandlungsliegenlänge benötigen als auch verhältnismäß kleine Patienten, bei der das Gerät mittels der langgestreckten Aussparungen am zum Kopfende entgegengesetzten Ende der Aussparungen justiert wird, gezielt behandelt werden können.

Auch im Hinblick auf das Behandlungsgerät liegt der Erfindung der Gedanke zugrunde, dieses auf verschiedene Kopfumfänge von Patienten anpassbar zu gestalten. Da es beispielsweise zur Strahlenbehandlung sinnvoll ist, den Kopfbereich kreisförmig durch eine Behandlungseinrichtung zu umgeben, ist neben einem Auflageteil, auf den der Kopf des Patienten während der Behandlung aufgelegt wird, mindestens ein Deckteil angebracht, das verschwenkbar angeordnet ist. Somit kann, bei geöffnetem Deckteil, der Kopf des Patienten nicht nur in Achsrichtung der zylinderförmigen Öffnung (im geschlossenen Zustand) des Behandlungsgerätes in dieses eingeführt werden. Vielmehr ist es auch möglich, den Kopf in natürlicher Weise von oben in das Behandlungsgerät einzulegen. Dennoch bleibt gewährleistet, dass im geschlossenen Zustand von Auflageteil und mindestens einem Deckteil der Kopf des Patienten umfasst wird, so dass eine wirkungsvolle Behandlung möglich bleibt.

Der erfindungsgemäßen Behandlungsvorrichtung liegt schließlich der Gedanke zugrunde, die Verstell- und somit Anpassmechanismen der Behandlungsliege und des Behandlungsgerätes zu einer Behandlungsvorrichtung zu integrieren, die auf die Bedürfnisse eines Patienten, insbesondere hinsichtlich seiner Körpergröße und seines Kopfumfanges gut anpassbar ist. Durch die Gestaltung der erfindungsgemäßen Behandlungsvorrichtung kann sich ein zu behandelnder Patient in gewöhnlicher Weise auf die Behandlungsliege setzen bzw. legen und seinen Kopf durch Absenken des Oberkörpers in das Behandlungsgerät einlegen. Ein kompliziertes Rutschen oder ähnliches ist nicht nötig.

Vorteilhafte Ausführungsformen sind durch die übrigen Ansprüche gekennzeichnet.

So umfasst die Behandlungsliege vorteilhafterweise ein schräg stellbares Kopfende. Dadurch kann der Patient in einer bequemen Sitzposition, die einer ergonomischen Ruheposition entspricht, behandelt werden. Dabei kann das Kopfende unveränderbar schräg gestellt sein, das heißt, dass die Behandlungsliege keine Position hat, in der der Patient tatsächlich liegt, oder aber die Schrägstellung des Kopfendes kann über einen geeigneten Schrägstellmechanismus wahlweise eingestellt werden und gegebenenfalls auch auf verschiedene Schrägstellungen justiert werden, so dass eine dem Patienten angenehme Position zur Behandlung gewählt werden kann. Dies ist insbesondere deshalb vorteilhaft, da der Patient eine verhältnismäßig lange Behandlungsdauer, etwa in der Größenordnung einer Stunde, auf der Liege je Behandlung verbringt. Zusätzlich ist zu bedenken, dass normalerweise mehrere Behandlungen in seitlich vorgegebenen Abständen durchgeführt werden müssen, so dass eine dem Patienten angenehme Position bei der Behandlung zum Wohlbefinden des Patienten deutlich beiträgt.

Dabei nimmt vorteilhafterweise das schräg stellbare Kopfende etwa die Hälfte der Länge der Behandlungsliege ein. Diese Aufteilung ist bevorzugt, da in diesem Fall das Gesäß und die Beine des Patienten in einer im Wesentlichen horizontalen Lage ausgerichtet sein können und der Rumpf- und Kopfbereich hingegen in einer schrägen Position von der Behandlungsliege unterstützt werden.

Ein einfacher Verstellmechanismus für die Behandlungsliege, insbesondere zur Schrägstellung des Kopfendes der Behandlungsliege kann durch eine Vorrichtung erzielt werden, wie sie beispielsweise von Deckstühlen bekannt. Der Tragkörper, der vorteilhafterweise mit den Beinen des Gestells der Behandlungsliege versehen ist, die auf dem Boden stehen, weist dabei mindestens einen Einrastbereich für ein am Kopfteil schwenkbar angebrachtes Stützteil auf. Durch Einrasten des Stützteils im Einrastbereich wird das Kopfteil in die Schrägstellung gebracht und durch das Stützteil in dieser Schrägstellung abgestützt. Durch Vorsehen mehrerer Einrastbereiche am Tragkörper, die beispielsweise stufenweise gestaltet sein können, wird eine Verstellung in mehrere Schrägstellungen bezüglich des im Wesentlichen horizontalen Fußendes des Gestells ermöglicht. In dieser Beschreibung bedeutet "Längsrichtung" durchwegs die Haupterstreckungsrichtung der Behandlungsliege, die der Kopf/Fußrichtung eines Patienten, wenn er auf der Behandlungsliege ruht, entspricht. Mit "Kopfende" ist derjenige Bereich der Behandlungsliege gemeint, in dem der Kopf eines Patienten während der Behandlung ruht, entsprechend ist mit "Fußende" der Bereich der Behandlungsliege gemeint, in dem die Füße des Patienten während der Behandlung sind.

Das Gestell der Behandlungsliege kann bevorzugterweise aus verschiedenen Materialien sein, etwa aus Holz oder Metall. Holz bietet den Vorteil eines ansprechenden Erscheinungsbildes, wohingegen Metall im Praxis- bzw. Klinikbetrieb sich aus Hygienegründen bewährt hat. Metall ist ferner durch eine hohe Robustheit gekennzeichnet.

Nach einer vorteilhaften Ausführungsform ist die Auflage eine weich gepolsterte Auflage, beispielsweise durch eine Schaumstoffeinlage, die mit einem entsprechenden Überzug versehen ist. Dies dient insbesondere dem Komfort des Patienten. Ferner kann durch die weiche Polsterung eine anatomische Formgebung der Auflage bewirkt werden, so dass die Körperbereiche des Patienten während der Behandlung durch die Behandlungsliege und insbesondere deren Auflage, optimal unterstützt werden. Dies führt wiederum dazu, dass der Patient das Empfinden des bequem Liegens bzw. Sitzens hat. Die Gestaltung der Auflage durch eine innere Polsterung und einen entsprechenden Überzug ist ebenfalls vorteilhaft, da im Falle des Verschmutzens der Überzug getrennt von der Polsterung einfach gereinigt werden kann.

Vorteilhafterweise umfasst die Auflage ein getrenntes Kopfteil und ein getrenntes Fußteil. Indem die Auflage mindestens zweiteilig gestaltet wird, wird sichergestellt, dass bei einem schräg stellbaren Gestell im Bereich des Knicks des Gestells keine für den Patienten unangenehmen Wulste in der Auflage entstehen. Selbstverständlich kann die Auflage auch aus mehr als zwei Teilen bestehen. Insbesondere können beispielsweise das Kopfteil und/oder das Fußteil ihrerseits wiederum mehrteilig gestaltet sein.

Ferner ist für die Behandlungsliege eine ergonomische Gestaltung bevorzugt. Diese wird zweckmäßig durch die Auflage erreicht. Im Falle der erwähnten gepolsterten Auflage kann die ergonomische Gestaltung der Behandlungsliege, und insbesondere der Auflage einfach durch eine entsprechende Gestaltung der Polsterung und des Überzuges erreicht werden. Aber auch bei anderen Auflagen, beispielsweise einem gespannten Stoff zwischen dem Gestell, entsprechend geformten Kunststoff, entsprechend geformtem Holz oder ähnlichem ist eine ergonomische Gestaltung möglich. Insbesondere ist es vorteilhaft, wenn die Auflage im Bereich, in dem das Gesäß eines Patienten ruht, eine Vertiefung gegenüber dem Bereich, in dem die Beine des Patienten ruhen, aufweist. Dadurch wird einerseits erreicht, dass die Beine des Patienten während der Behandlung im Vergleich zum Gesäß etwas erhöht liegen, was vorteilhaft für die Blutzirkulation ist und somit wiederum zum Wohlbefinden es Patienten beiträgt, und andererseits wird durch die Vertiefung eine vorgegebene Position des Patienten auf der Behandlungsliege erreicht. Dies ist sinnvoll, da dadurch verhindert werden kann, dass der Patient während der Behandlung seine Position wesentlich ändert und somit beispielsweise eine Bestrahlung mit einem vorab eingestellten Bestrahlungsgerät auf die falschen Körperteile einwirkt. Außerdem ermöglicht diese Gestaltung einen reduzierten Kontrollaufwand durch Personal, da die Wahrscheinlichkeit, dass der Patient seine Position ändert, verringert ist.

Bevorzugterweise ist auch der die Lendengegend untersützende Bereich erhaben gegenüber dem Bereich, in dem das Behandlungsgerät eingebracht wird, ausgeführt. Dies dient insbesondere dem Einhalten der korrekten Behandlungsposition, da der Patient unmittelbar bei einer unerwünschten Änderung seiner Position bemerkt, dass seine Lage auf der Behandlungsliege nicht mehr korrekt ist, da er das Gefühl der Unbequemlichkeit erfährt.

Aus ästhetischen Gründen wird es bevorzugt, dass die Auflage im Bereich der mindestens einen Aussparung im Gestell eine Aussparung aufweist, die diese im Gestell vorhandene Aussparung freilegt. Dadurch wird ein blockierungsfreies und einfaches Betätigen des Verstellmechanismus des Behandlungsgerätes gewährleistet. Aus ästhetischen Gründen wird es bevorzugt, dass diese Aussparung in der Auflage durch ein oder mehrere Füllstücke abdeckbar ist. Dies ist hinsichtlich der Funktionalität nicht zwingend erforderlich, bietet jedoch ein ansprechenderes Erscheinungsbild, wenn der Eindruck entsteht, dass keine Lücken in der Auflage vorhanden sind.

Bevorzugterweise ist ein Verstellmechanismus zur Verstellung eines Behandlungsgerätes für den Kopfbereich eines Patienten vorgesehen. Dabei operiert der Verstellmechanismus so, dass er das Behandlungsgerät in Längsrichtung, also in Kopf/Fußrichtung des Patienten, verschieben kann.

Der Verstellmechanismus umfasst vorteilhafterweise in einer kostengünstigen Ausführungsform eine Platte, die an der der Auflageseite des Gestells abgewandten Seite befestigt ist. Diese Befestigung kann beispielsweise über Schrauben vorgenommen werden. Alternative Befestigungsmittel, beispielsweise eine Klebeverbindung oder ähnliches, sind ebenfalls denkbar. An dieser Platte ist verschiebbar mindestens ein Träger für das Behandlungsgerät angebracht, der vorteilhafterweise durch die entsprechenden Aussparungen im Gestell sowie der Auflage, die gewöhnlich auf dem Gestell liegt, reicht. Daraus ergibt sich ferner, dass nach einer bevorzugten Ausführungsform die langgestreckten Aussparungen im Gestell so geformt sind, dass sie gerade den Träger bzw. die Träger für das Behandlungsgerät durchlassen und bei einer Verschiebung der Träger diese reibungsfrei ermöglichen. Eine übermäßige Breite der Aussparungen wird nicht bevorzugt, da in diesem Fall die Stabilität und Steifigkeit des Gestells verringert würde.

Nach einer bevorzugten Ausführungsform ist der mindestens eine Träger durch eine Spindel und eine Kurbel zur Betätigung der Spindel verschiebbar. Dies ist ein zuverlässiger Verschiebemechanismus, der einfach zu betätigen und genau auf die Körperlänge des Patienten zu justieren ist.

Nach einer bevorzugten Ausführungsform weist die Behandlungsliege ferner eine Aufnahme für eine Steuer- und/oder Signalerzeugungsgerät für ein Behandlungsgerät auf. Durch eine solche Aufnahme, die beispielsweise in der Form eines offenen Quaders an der Behandlungsliege angebracht sein kann, etwa in der von deren Fußteil, bietet eine kompakte Unterbringung des Steuer- und Signalerzeugungsgerätes an der Behandlungsliege, so dass ein zusätzliches Gestell zur Aufnahme dieses Gerätes nicht nötig ist. Dies trägt wiederum zu einem ansprechenden Erscheinungsbild der gesamten Vorrichtung bei. Die Aufnahme für das Steuer- und/oder Signalerzeugungsgerät ist dabei selbstverständlich vorteilhafterweise so gestaltet, dass das Gerät durch eine Bedienperson direkt und einfach zugänglich ist.

Das Behandlungsgerät weist nach einer bevorzugten Ausführungsform zwei Deckteile auf, wobei an jedem freien Ende des Auflageteils jeweils ein Deckteil schwenkbar angebracht ist. Dadurch wird einerseits eine genaue Justierung des Behandlungsgerätes um den Kopf des Patienten möglich, andererseits kann der Patient seinen Kopf einfach in das Behandlungsgerät einbringen. Im Gegensatz zum Vorsehen von nur einem Deckteil muss ferner der Schwenkwinkel beim Vorsehen von zwei Deckteilen für jedes Deckteil nicht so groß sein, wie es im Fall eines Deckteils nötig ist, was wiederum das Anbringen von Signalübertragungseinrichtungen zwischen dem Auflageteil und den Deckteilen vereinfacht. Selbstverständlich können auch mehr als zwei Deckteile vorgesehen werden, wobei in diesem Fall jeweils weitere Deckteile an entsprechenden anderen Deckteilen in ähnlicher Weise schwenkbar angebracht sind. Auch müssen in diesem Fall die Signalübertragungseinrichtungen zwischen den entsprechenden Deckteilen vorgesehen werden.

Die Deckteile und das Auflageteil ergänzen sich im geschlossenen Zustand vorteilhafterweise zu einem Ring. Dadurch kann der Kopf des Patienten durch einen nahezu geschlossenen Ring umfasst werden, so dass eine gezielte Behandlung möglich ist. Auch wenn der Patient seinen Kopf in dem Behandlungsgerät dreht, bleibt die Ausrichtung der Behandlung auf den Kopf gewährleistet.

Als Verschwenkeinrichtung wird bevorzugterweise ein plattenförmiges Element mit zwei Hauptseiten verwendet, wobei eine Dreheinrichtung für eine Relativdrehung zwischen dem Auflageteil und dem plattenförmigen Element und eine Dreheinrichtung für eine Relativdrehung zwischen dem plattenförmigen Element und dem Deckteil vorgesehen ist. Das plattenförmige Element wird dabei so an dem Auflageteil angebracht, dass es mit einer seiner Hauptflächen an einer der Stirnflächen des plattenförmigen Elementes anliegt. Durch diese einfache und zuverlässige Verschwenkeinrichtung, die beispielsweise durch Bolzen als Dreheinrichtung realisiert werden kann, wird ein Verschwenken zwischen Auflageteil und Deckteilen möglich, wobei gleichzeitig eine gute Justierung der Teile zueinander und zum Kopf des Patienten erhalten bleibt.

Dabei ist vorteilhafterweise das Behandlungsgerät so gestaltet, dass die Verschwenkeinrichtung von Hand betätigt werden kann, aber an sich einen solchen Reibungswiderstand aufweist, dass sie ohne Krafteinwirkung in der eingestellten Position verbleibt. Damit sind keine zusätzlichen Geräte oder Werkzeuge zum Justieren des Behandlungsgerätes um den Kopf des Patienten nötig.

Vorteilhafterweise ist das Auflageteil jeweils auf einer der Hauptflächen des plattenförmigen Elementes angebracht, wohingegen das oder die Deckteile auf der anderen Hauptfläche des plattenförmigen Elementes vorgesehen sind. Dies dient wiederum einer gezielten Anordnung des Behandlungsgerätes um den Kopf des Patienten.

Diese Anordnung ist insbesondere dann vorteilhaft, wenn das Behandlungsgerät ein Magnetfeld-Therapiegerät ist. Durch ein Magnetfeld-Therapiegerät können Felder mit bestimmten Signalen, wie pulsierende Felder, die über ein Magnetfeld aufgebracht werden, gezielt auf den Kopf des Patienten gelenkt werden. Dies hat sich insbesondere für die Tinnitusbehandlung bewährt.

### Kurze Beschreibung der Zeichnungen

Nachfolgend wird die Erfindung rein beispielhaft anhand der angefügten Figuren beschrieben, in denen:
- Fig. 1: eine perspektivische Ansicht einer erfindungsgemäßen Bearbeitungsliege ist;
- Fig. 2: eine perspektivische Ansicht von hinten auf eine Ausführungsform der erfindungsgemäßen Behandlungsliege ist;
- Fig. 3: eine Ansicht von vorne auf eine Ausführungsform der erfindungsgemäßen Behandlungsliege ist;
- Fig. 4: eine perspektivische Ansicht auf ein Behandlungsgerät gemäß einer Ausführungsform gemäß der Erfindung ist, wobei das Behandlungsgerät an einer erfindungsgemäßen Liege angebracht ist; und
- Fig. 5: eine Draufsicht auf ein erfindungsgemäßes Behandlungsgerät im in einer erfindungsgemäßen Behandlungsliege eingebauten Zustand ist.

### Wege zur Ausführung der Erfindung

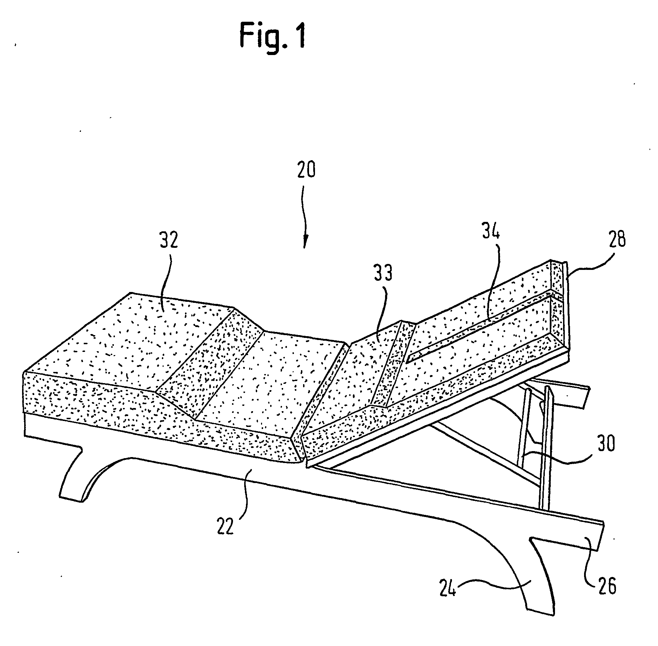

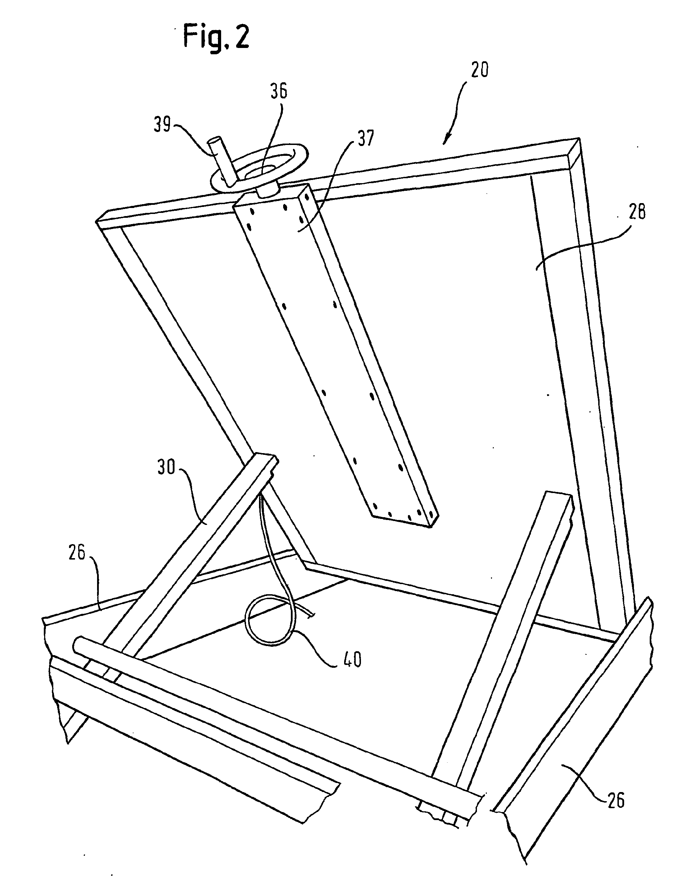

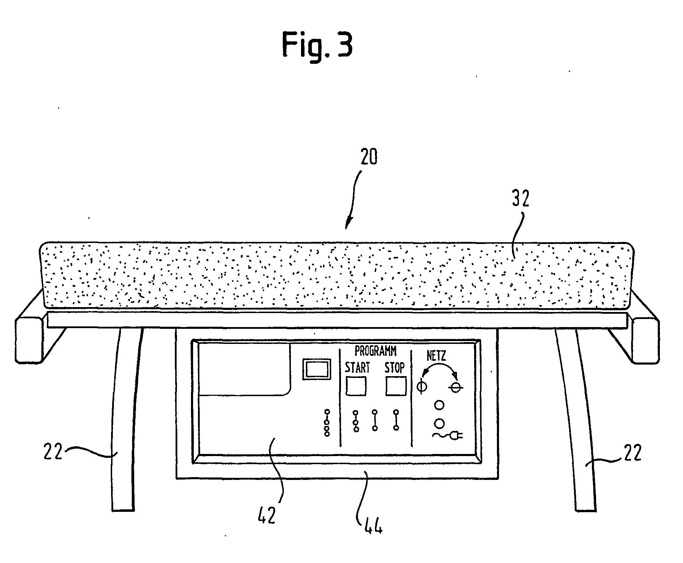

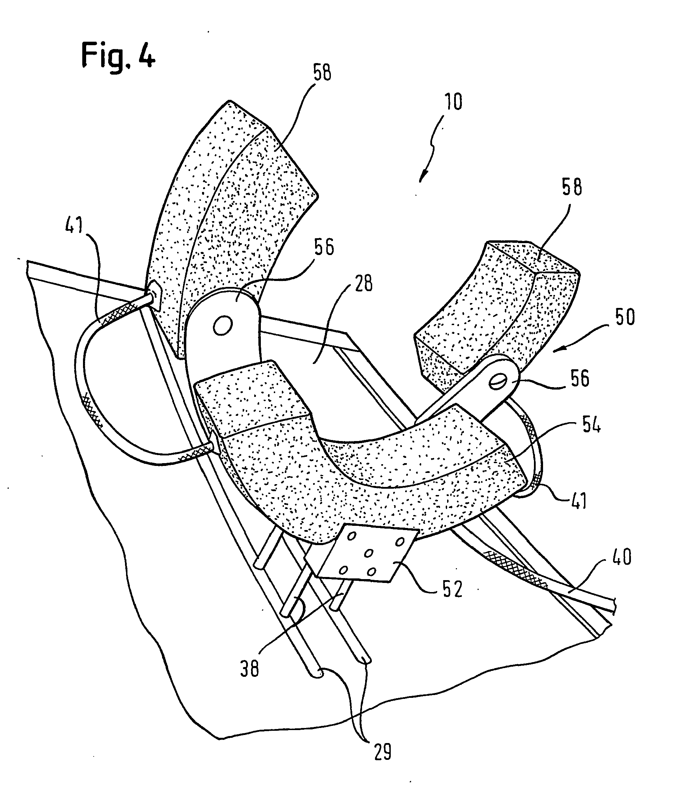

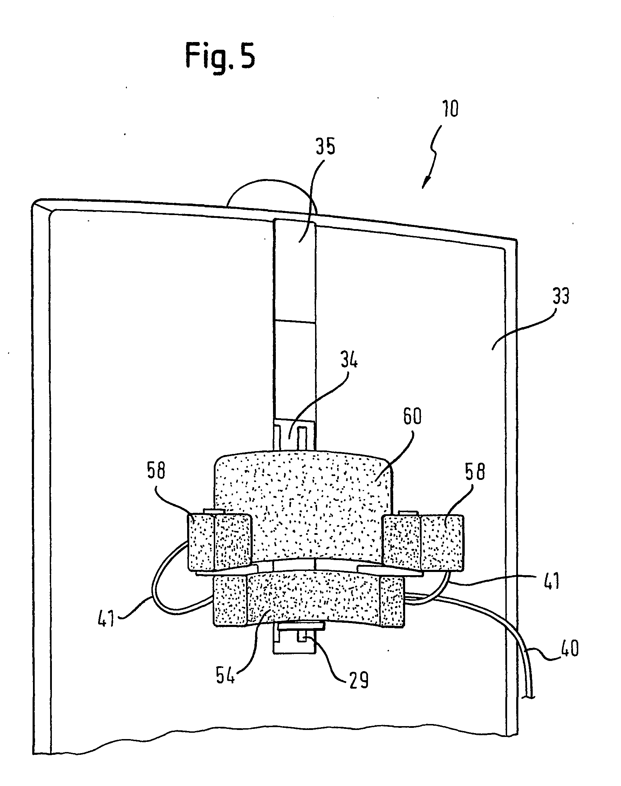

Fig. 1 zeigt in perspektivischer Ansicht eine Behandlungsliege 20, die einen Teil einer erfindungsgemäßen Behandlungsvorrichtung 10 bildet, die beispielsweise in Fig. 4 und 5 im Ausschnitt gezeigt ist. Fig. 2 und 3 zeigen jeweils eine perspektivische Ansicht von hinten auf die Behandlungsliege bzw. eine Ansicht von vorne auf die Behandlungsliege.

In Fig. 1 ist zu erkennen, dass die Behandlungsliege 20 im Wesentlichen ein Gestell 22 umfasst, das mit Füßen 24 versehen ist. Mit den Füßen 24 steht die Behandlungsliege etwa in der Form eines gewöhnlichen Bettes auf dem Boden. Die Höhe der Behandlungsliege 20 ist vorteilhafterweise dabei so gewählt, dass sich ein Patient ohne weiteres auf die Behandlungsliege mit aufgebrachter Auflage setzen kann. Die Abmessungen in Längsrichtung und Breitenrichtung der Behandlungsliege 20 entsprechen dabei Abmessungen eines gewöhnlichen Bettes, jedoch in jedem Fall sollten die Abmessungen so bemessen sein, dass ein Patient bequem auf der Behandlungsliege 20 Platz nehmen kann.

Das Gestell 22 umfasst neben dem sich im Wesentlichen horizontal erstreckenden Tragkörper 26 mit den daran angebrachten Füßen 24 ein Kopfteil 28. Dieses Kopfteil ist aus der Horizontalen heraus schräggestellt, d.h. es ist ein Winkel größer 0° zwischen dem sich im Wesentlichen horizontal erstreckenden Tragkörper 26 des Gestells 22 und dem Kopfteil 28 des Gestells 22 vorhanden. Je nach dem, ob das Gestell der Behandlungsliege einen Verstellmechanismus für die Schrägstellung des Kopfteils 28 aufweist, kann dieser Winkel entsprechend den Patientenwünschen in gewissem Bereich eingestellt werden. In der in der Fig. 1 dargestellten Ausführungsform ist dazu ein Stützteil 30 vorgesehen, das im sich horizontal erstreckenden Tragkörper in verschiedenen Positionen einrasten kann. Am Kopfteil 28 ist dieses Stützteil 30 über ein Gelenk befestigt. Somit kann durch eine unterschiedliche Einrastung des Stützteils 30 in dem Tragkörper 26 des Gestells 22 eine gewünschte Neigung des Kopfteils 28 des Gestells 22 eingestellt werden. Selbstverständlich ist es alternativ dazu möglich, die Behandlungsliege so zu gestalten, dass die Neigung des Kopfteils fest vorgegeben ist und nicht verändert werden kann. Die Auflagefläche des Gestells der Behandlungsliege kann im Wesentlichen frei gestaltet werden. Es kann beispielsweise eine plattenförmige, durchgängige Unterstützung für die Auflage bzw. Auflagen 32, 33 der Behandlungsliege vorgesehen werden. Alternativ dazu können auch Verstrebungen, also keine durchgängige Platte, vorgesehen werden, die die Auflagen 32, 33 unterstützen.

Das Material, aus dem das Gestell der Behandlungsliege gefertigt ist, ist ebenfalls nahezu frei wählbar. Holz bietet beispielsweise ein optisch ansprechendes Erscheinungsbild. Wenn die Behandlungsliege 20 beispielsweise zur Tinnitusbehandlung eingesetzt wird, ist mit keiner nennenswerten Verschmutzung zu rechnen und die Hygieneanforderungen sind ebenfalls nicht besonders erhöht, so dass in diesem Fall Holz geeignet ist. Alternativ dazu ist es selbstverständlich möglich, das Gestell 22 der Behandlungsliege aus einem anderen Material aufzubauen, etwa Metall.

Die Behandlungsliege 20 umfasst ferner Auflagen 32, 33. In der in Fig. 1 dargestellten Ausführungsform sind dies gepolsterte Auflagen, die beispielsweise aus einem Schaumstoffkörper mit Überzug bestehen. Für den Komfort des Patienten ist es angenehm, wenn die Auflagen 32, 33 in gewissem Maß nachgiebig gestaltet sind.

Wie in Fig. 1 deutlich zu erkennen ist, sind die Auflagen 32, 33 ergonomisch geformt. Insbesondere bedeutet dies, dass die im Beinbereich des Patienten angebrachte Auflage 32 eine Vertiefung für das Gesäß des Patienten aufweist. In der in Fig. 1 dargestellten Ausführungsform ist diese Vertiefung über die Breitenrichtung der Auflage unverändert. Selbstverständlich kann die Vertiefung jedoch, wenn dies erforderlich erscheint, auch in der Breitenrichtung veränderlich gestaltet werden. Die Vertiefung für das Gesäß im Gegensatz zu der erhöhten Position für die Beine stellt sicher, dass die Beine des Patienten während der Behandlung hochgelagert werden, was durch eine günstige Beeinflussung der Blutzirkulation dem Wohlbefinden des Patienten entgegenkommt.

Die im Kopf- und Rumpfbereich es Patienten vorgesehene Auflage 33 grenzt in gleicher Höhe an die Auflage 32 an, so dass im Übergangsbereich kein Wulst oder Ähnliches entsteht. Zur Vermeidung von Wulsten ist es auch vorteilhaft, die Auflage 32, 33, insbesondere bei einem schräg gestellten Kopfteil 28 der Behandlungsliege, zweiteilig zu gestalten, da andernfalls Wulste im Übergangsbereich hervorgerufen würden. Die im Kopf-/Rumpfbereich vorgesehene Auflage 33 weist ferner zum Kopfbereich hin ebenfalls eine Vertiefung gegenüber dem den Lendenbereich unterstützenden Bereich auf. Diese Vertiefung dient insbesondere dazu, dem Patienten ein Verrutschen aus der korrekten Behandlungsposition rasch zu signalisieren. Zusätzlich erleichtert sie das Einbringen des Behandlungsgerätes, das durch die in dem Gestell 22 und in der Auflage 33 vorgesehene Aussparungen über Träger geführt wird.

Wie es aus Fig. 5 zu erkennen ist, ist zur Führung und zur Sicherstellung der Verschiebung des Behandlungsgerätes in der Auflage 33 eine Aussparung 34 geformt. Diese Aussparung 34 legt die in dieser Ausführungsform doppelt vorhandenen, in Längsrichtung des Gestells und insbesondere dessen Kopfteils 28 verlaufenden, langgestreckten Aussparungen 29 frei, durch die Träger für das Behandlungsgerät geführt werden (siehe Fig. 4; die Darstellung der Fig. 4 ist ohne Auflage 33). Aus Fig. 5 ist ferner zu erkennen, dass die Aussparung 34 in der Auflage 33 zumindest teilweise durch Einsätze 35 abgedeckt sein kann. Diese Einsätze 35 dienen insbesondere dem Sicherstellen eines ansprechenden Erscheinungsbildes.

Fig. 2 zeigt eine perspektivische Ansicht von hinten von der Behandlungsliege. Neben den bereits in Verbindung mit Fig. 1 beschriebenen Elementen ist in dieser Darstellung deutlich der Verstellmechanismus 36 für Träger 38 (siehe Fig. 4) eines Behandlungsgerätes zu erkennen. Der Verstellmechanismus ist an der rückwärtigen Seite des Kopfteils 28, also der Seite, die der Auflageseite abgewandt ist, über Schrauben oder ähnliches befestigt. Insbesondere wird eine Platte 37 über die Aussparung 34 im Kopfteil 28 des Gestells 22 befestigt, die ihrerseits den über eine Kurbel 39 betätigbaren Verstellmechanismus, beispielsweise in Form einer Spindel, für die Träger 38 beinhaltet. Das plattenförmige Element 37 ist dabei so angeordnet, dass die Träger 38 in den dafür vorgesehenen Aussparungen 29 und 34 im Gestell und der Auflage 33 verlaufen. Selbstverständlich sind auch andere Verschiebemechanismen für die Träger 38 möglich. Die Befestigung der Platte 37, die in der dargestellten Ausführungsform über Schrauben am Kopfteil 28 vorgenommen wird, kann selbstverständlich auch anders gestaltet werden.

Zusätzlich ist in Fig. 2 ein Kabel 40 zu erkennen, das von einem Behandlungsgerät zu einer Signalerzeugungs- und/oder Steuereinheit läuft. Dabei wird dieses Kabel 40 vorzugsweise auf der Unterseite der Behandlungsliege geführt, so dass es für Patient und Bedienpersonal keine Stolpergefahr darstellt.

Fig. 3 zeigt eine Ansicht der Behandlungsliege von vorne. Es ist zu erkennen, dass ein Steuer- und/oder Signalerzeugungsgerät 42 in eine dafür vorgesehene kantenförmige Aufnahme 44 am Behandlungsgerät eingesetzt ist. Die Aufnahme 44 ist dabei hinsichtlich der Größe dem Signalerzeugungs- und Steuergerät angepasst. Zusätzlich ist sie so angebracht, dass ein Bediener sie ohne Probleme erreichen kann. Selbstverständlich ist die Anbringung nicht auf die dargestellte Position am Gestell 22 beschränkt. Es kann jede beliebige Position am Gestell 22 gewählt werden, so lange eine gute Zugänglichkeit gewährleistet bleibt.

Fig. 4 und 5 zeigen eine Behandlungsvorrichtung 10, die neben der in Verbindung mit Fig. 1 bis 3 beschriebenen Behandlungsliege ein Behandlungsgerät 50 umfasst. Das Behandlungsgerät 50 ist über eine Befestigungsvorrichtung 52, im dargestellten Fall eine einfache Schraubenverbindung und eine Platte, mit den Trägern 38, die die Verbindung zum Verstellmechanismus in Längsrichtung entlang der Behandlungsliege bilden, versehen. In der dargestellten Ausführungsform sind vier Träger 38 vorgesehen. Selbstverständlich ist es möglich, mehr als vier oder weniger als vier solcher Träger vorzusehen, solange die Stütz- und Verschiebefunktion über den Verstellmechanismus sichergestellt bleibt. Als Befestigungsvorrichtung 52 des Behandlungsgerätes 50 an den Trägern 38 sind ebenfalls verschiedene Möglichkeiten neben der dargestellten denkbar.

Das Behandlungsgerät 50 in der dargestellten Ausführungsform umfasst ferner ein Auflageteil 54, das als Ringsegment gestaltet ist. Dabei ist das Ringsegment so ausgerichtet, dass es im Wesentlichen die Nackenform eines Patienten abbildet. Das Ringsegment 54 ist vorteilhafterweise aus einer nachgiebigen Auflage, so dass der Kopf des Patienten bzw. der Nacken weich ruht, wenn er in der Auflage liegt. An den freien Enden des Auflageteils 54 sind plattenförmige Elemente 56 vorgesehen, die die Verbindung zu Deckteilen 58, die ebenfalls weich gestaltet sind, um Verletzungen zu vermeiden, vorhanden. Die Verbindungen zwischen dem Auflageteil 54 und den plattenförmigen Elementen 56 bzw. den plattenförmigen Elementen 56 und den Deckteilen 58, von denen in der dargestellten Ausführungsform zwei vorhanden sind, ist jeweils so, dass das plattenförmige Element 56 gegen das jeweils andere Element verschwenkt werden kann. Dies bedeutet, dass im geschlossenen Zustand das Auflageteil 54 und die Deckteile 58 eine im Wesentlichen ringförmige Konfiguration mit nur sehr kleinen Zwischenräumen oder ohne Zwischenräume bilden. Die plattenförmigen Elemente 56 sind vorteilhafterweise so vorgesehen, dass die gewünschten Schwenkstellungen zwischen Auflageteil 54 und Deckteil 58 von Hand eingestellt werden können. Dennoch sollten die eingestellten Positionen dieser Teile sich durch die Reibung zwischen den plattenförmigen Elementen 56 und dem Auflageteil 54 bzw. den Deckteilen 58 nicht ohne äußere Krafteinwirkung verändern, so dass das Behandlungsgerät in einer stabilen Position bleibt.

Aus Fig. 4 ist ferner deutlich zu erkennen, dass eine signalübertragende Verbindung durch das Kabel 40 zunächst von einer externen Steuer- und/oder Signalerzeugungseinheit zum Behandlungsgerät 50 und innerhalb des Behandlungsgerätes über die Kabel 41 zwischen dem Auflageteil 54 und den Deckteilen 58 vorhanden ist.

Damit der gesamte Kopf des Patienten bequem während der Behandlung ruht, können zusätzliche Auflagen, beispielsweise wie die in Fig. 5 dargestellte Auflage 60 vorgesehen werden.

Alle mit dem Körper des Patienten in unmittelbare Berührung kommenden Teile sind vorzugsweise nachgiebig gestaltet, beispielsweise aus Schaumstoffmaterial.

Der wesentliche Aspekt der Erfindung liegt darin, eine beispielsweise zur Tinnitusbehandlung über Magnetfeldtherapie auf die individuellen Bedürfnisse anpassbare Behandlungsvorrichtung zu bilden, die im Wesentlichen eine Behandlungsliege sowie ein Behandlungsgerät umfassen.

## Patentansprüche

1. Behandlungsvorrichtung (10), umfassend
- eine Behandlungsliege (20), mit einem Gestell (22) sowie mindestens einer auf oder an dem Gestell (22) platzierbaren Auflage (32, 33), wobei in dem Gestell und der Auflage mindestens eine in Längsrichtung des Gestells verlaufende, langgestreckte Aussparung (29, 34) vorgesehen ist, die sich ausgehend von einem Kopfende der Behandlungsliege (20) in einem in Breitenrichtung mittleren Bereich erstreckt,
- ein Behandlungsgerät (50) mit einem Auflageteil für den Kopf eines Patienten, wobei das Auflageteil im Wesentlichen die Form eines Ringsegments aufweist; mindestens einem über eine Verschwenkeinrichtung (56) an mindestens einem freien Ende des Auflageteils angebrachtes Deckteil, wobei das mindestens eine Deckteil (58) mit dem Auflageteil über eine Signalübertragungseinrichtung (41) verbunden ist; einer Befestigungseinrichtung (52) für das Behandlungsgerät; und einem mit dem Behandlungsgerät verbundenes Signalerzeugungs- und/oder Steuergerät (42),
- wobei die Behandlungsliege einem Verstellmechanismus (36) zur Verstellung des Behandlungsgeräts in Längsrichtung umfasst; und
- wobei die Behandlungsvorrichtung mindestens einen Träger (38) für das Behandlungsgerät (50) umfasst, der durch die langgestreckte Aussparung geführt wird und die Verbindung zum Verstellmechanismus bildet;

2. Behandlungsvorrichtung (10) nach Anspruch 1,
**dadurch gekennzeichnet, dass** zwei Deckteile (58) vorgesehen sind, wobei an jedem freien Ende des Auflageteils (54) jeweils ein Deckteil (58) schwenkbar angebracht ist.

3. Behandlungsvorrichtung (10) nach Anspruch 1 oder 2, **dadurch gekennzeichnet, dass** sich das Auflageteil (54) und das mindestens eine Deckteil (58) zu einem Ring ergänzen.

4. Behandlungsvorrichtung (10) nach einem der Ansprüche 1 bis 3,
**dadurch gekennzeichnet, dass** die Verschwenkeinrichtung ein plattenförmiges Element (56) umfasst, an dem eine Dreheinrichtung für eine Relativdrehung zwischen Auflageteil (54) und plattenförmigem Element (56) und eine Dreheinrichtung zwischen plattenförmigem Element (36) und Deckteil (58) vorgesehen ist.

5. Behandlungsvorrichtung (10) nach einem der vorhergehenden Ansprüche,
**dadurch gekennzeichnet, dass** das Auflageteil (54) auf einer Hauptfläche des plattenförmigen Elements (56) und das mindestens eine Deckteil (58) auf der anderen Hauptfläche des plattenförmigen Elements (56) vorgesehen ist.

6. Behandlungsvorrichtung (10) einem der vorhergehenden Ansprüche,
**dadurch gekennzeichnet, dass** das Behandlungsgerät ein Magnetfeld-therapiegerät ist.

7. Behandlungsvorrichtung (10) nach einem der vorhergehenden Ansprüche,
**dadurch gekennzeichnet, dass** das Gestell (22) ein schrägstellbares Kopfende umfasst.

8. Behandlungsvorrichtung (10) nach Anspruch 7,
**dadurch gekennzeichnet, dass** das schrägstellbare Kopfende etwa die Hälfte der Länge der Behandlungsliege einnimmt.

9. Behandlungsvorrichtung (10) nach einem der vorhergehenden Ansprüche,
**dadurch gekennzeichnet, dass** das Gestell einen sich im Wesentlichen horizontal erstreckenden Tragkörper (26), ein im in Längsrichtung mittleren Bereich des Tragkörpers schwenkbar angebrachtes Kopfteil (28) und ein gelenkig am Kopfteil angebrachtes Stützteil (30) umfasst, das in einen am Tragkörper (26) vorgesehenen Einrastbereich mit seinem freien Ende einrasten kann, um eine Schrägstellung des Kopfteils (28) zu bewirken.

10. Behandlungsvorrichtung (10) nach Anspruch 9,
**dadurch gekennzeichnet, dass** mehrere Einrastbereiche am Tragkörper (26) vorgesehen sind, so dass das Kopfteil verschiedene Schrägstellungen einnehmen kann.

11. Behandlungsvorrichtung (10) nach einem der vorhergehenden Ansprüche,
**dadurch gekennzeichnet, dass** das Gestell (22) aus Holz ist.

12. Behandlungsvorrichtung (10) nach einem der Ansprüche 1-10,
**dadurch gekennzeichnet, dass** das Gestell (22) aus Metall ist.

13. Behandlungsvorrichtung (10) nach einem der vorhergehenden Ansprüche,
**dadurch gekennzeichnet, dass** die mindestens eine Auflage (32, 33) eine weich gepolsterte Auflage ist.

14. Behandlungsvorrichtung (10) nach einem der vorhergehenden Ansprüche,
**dadurch gekennzeichnet, dass** die Auflage ein getrenntes Kopfteil (33) und ein Fußteil (32) umfasst.

15. Behandlungsvorrichtung (10) nach einem der vorhergehenden Ansprüche,
**dadurch gekennzeichnet, dass** die Auflage (32) im Bereich, in dem das Gesäß eines Patienten ruht, eine Vertiefung aufweist und im Bereich, in dem die Beine ruhen, demgegenüber erhaben ist.

16. Behandlungsvorrichtung (10) nach einem der vorhergehenden Ansprüche,
**dadurch gekennzeichnet, dass** die Auflage (32) im Bereich der mindestens einen Aussparung im Gestell eine Aussparung (34) aufweist, die mindestens die im Gestell vorhandene Aussparung (29) freilegt, und dass für die Aussparung in der Auflage mindestens ein Füllstück (35) vorgesehen ist, um die Aussparung (34) zumindest teilweise abzudecken.

17. Behandlungsvorrichtung (10) nach einem der vorhergehenden Ansprüche,
**dadurch gekennzeichnet, dass** die Auflage (33) im Bereich der Aussparung (34) vertieft gegenüber einem erhabenen Bereich zur Abstützung der Lendengegend eines Patienten gestaltet ist.

18. Behandlunasvorrichtung_(10) nach einem der vorhergehenden Ansprüche,,
**dadurch gekennzeichnet, dass** der Verstellmechanismus (36) eine an der der Auflageseite abgewandten Seite befestigte Platte (37) umfasst, an der verschiebbar der mindestens eine Träger (38) für das Behandlungsgerät angebracht ist.

19. Behandlungsvorrichtung (10) nach einem der vorhergehenden Ansprüche,
**dadurch gekennzeichnet, dass** der mindestens eine Träger (38) durch eine Spindel und eine Kurbel (39) zur Betätigung der Spindel verschiebbar ist.

20. Behandlungsvorrichtung (10) nach einem der vorhergehenden Ansprüche,
**dadurch gekennzeichnet, dass** eine Aufnahme (44) für eine Signalerzeugung und/oder Steuergerät (42) für ein Behandlungsgerät vorgesehen ist.

## Claims

1. Treatment device (10) comprising
- a treatment bed (20) having a frame (22) and at least one bearing element (32, 33) which can be placed on the frame (22), wherein at least one elongated recess (29, 34) extending in the longitudinal direction of the frame is provided in the frame and the bearing element, which extends from a head section of the treatment bed (20) into a central area in the width wise direction,
- a treatment apparatus (50) comprising a bearing part for the head of a patient, said bearing part essentially taking the shape of a ring segment; at least one cover part placed on at least one free end of the bearing part by means of a pivoting device (56) with the at least one cover part (58) being connected to the bearing part by means of a signal transmission device (41); a fixing device (52) for the treatment apparatus; and a signal generating device and/or control device (42) connected to the treatment apparatus,
- wherein the treatment bed comprises an adjustment mechanism (36) for adjusting the treatment apparatus in the longitudinal direction; and
- wherein the treatment device comprises at least one support (38) for the treatment apparatus which is guided through the elongated recess and forms the connection to the adjustment mechanism.

2. Treatment device (10) according to claim 1 **characterised in that** two cover parts (58) are provided, with one cover part (58) being pivotably attached to each free end of the bearing part (54).

3. Treatment device (10) according to claim 1 or 2, **characterised in that** the bearing part (54) and the at least one cover part (58) complete to form a ring.

4. Treatment device (10) according to one of claims 1 to 3, **characterised in that** pivoting device comprises a plate-like element (56) on which a rotating means for a relative rotation between the bearing part (54) and the plate-like element (56) and a rotating means between the plate-like element (56) and the cover part (58) is provided.

5. Treatment device (10) according to one of the preceding claims, **characterised in that** the bearing part (54) is provided on a main surface of the plate-like element (56) and the a least one cover part (58) is provided on the other main surface of the plate-like element (56).

6. Treatment device (10) according to one of the preceding claims, **characterised in that** said treatment apparatus is a magnetic field therapy device.

7. Treatment device (10) according to one of the preceding claims, **characterised in that** the frame (22) comprises an inclinable head section.

8. Treatment device (10) according to claim 7, **characterised in that** the inclinable head section takes up approximately half of the length of the treatment bed.

9. Treatment device (10) according to one of the preceding claims, **characterised in that** the frame comprises a supporting body (26) which extends basically horizontally, a head part (28) which is pivotably attached in the central area of the supporting body in the longitudinal direction, and a supporting part (30) attached to the head part in a flexible manner, which can be engaged with its free end in an engagement area provided on the supporting body (26) in order to cause an inclination of the head part (28).

10. Treatment device (10) according to claim 9, **characterised in that** several engagement areas are provided on the supporting body (26) so that the head part can assume different inclinations.

11. Treatment device (10) according to one of the preceding claims, **characterised in that** the frame (22) is made of wood.

12. Treatment device (10) according to claims 1 to 10, **characterised in that** the frame (22) is made of metal.

13. Treatment device (10) according to any of the preceding claims, **characterised in that** the at least one bearing element (32, 33) is a softly padded bearing element.

14. Treatment device (10) according to any of the preceding claims, **characterised in that** the bearing element comprises a separate head part (33) and foot part (32).

15. Treatment device (10) according to any of the preceding claims, **characterised in that** the bearing element (32) has a depression in the area in which the buttocks of a patient rest, whereas the area in which the legs rest is raised.

16. Treatment device (10) according to any of the preceding claims, **characterised in that** the bearing element (32) comprises a recess (34) in the area of the at least one recess in the frame, which exposes at least the excess (29) provided in the frame, and **in that** at least one filling piece (35) is provided for the recess in the bearing element in order to cover the recess (34) at least partially.

17. Treatment device (10) according to any of the preceding claims, **characterised in that** the bearing element (33) is designed so as to be depressed in the area of the recess (34) as compared to a raised area for supporting the patient's lumbar region.

18. Treatment device (10) according to any of the preceding claims, **characterised in that** the adjustment mechanism (36) comprises a plate (37) fixed on the side facing away from the bearing element side, to which the at least one support (38) for the treatment apparatus is attached in a shiftable manner.

19. Treatment device (10) according to any of the preceding claims, **characterised in that** the at least one support (38) is shiftable by means of a spindle and a crank (39) for operating the spindle.

20. Treatment device (10) according to any of the preceding claims, **characterised in that** a receptacle (44) for a signal generating device and/or control device (42) for a treatment apparatus is provided.

## Revendications

1. Dispositif de traitement (10) comprenant
- un lit de traitement (20) comportant un bâti (22) ainsi qu'au moins une surface portante (32, 33) pouvant être placée sur ou contre le bâti (22), où est prévu dans le bâti et la surface portante au moins un évidement (29, 34) allongé qui s'étend dans le sens longitudinal du bâti et qui, en partant d'une extrémité de tête du lit de traitement (20) s'étend dans une zone intermédiaire dans le sens de la largeur,
- un appareil de traitement (50) comportant une partie de soutien pour la tête d'un patient, la partie de soutien présentant essentiellement la forme d'un segment annulaire ; au moins une partie de recouvrement pouvant être appliquée par un dispositif de pivotement (56) au niveau d'une extrémité libre de la partie de soutien, la partie de recouvrement (58), au moins au nombre de une, étant reliée avec la partie de soutien via un dispositif de transmission de signal (41) ; un dispositif de fixation (52) pour l'appareil de traitement ; et un appareil de génération de signaux et/ou un appareil de commande (42) relié à l'appareil de traitement,
- où le lit de traitement comporte un mécanisme de réglage (36) permettant d'ajuster l'appareil de traitement dans le sens longitudinal ; et
- où le dispositif de traitement comporte au moins un support (38) pour l'appareil de traitement (50), qui est guidé par l'évidement allongé et forme la liaison vers le mécanisme de réglage.

2. Dispositif de traitement (10) selon la revendication 1, **caractérisé en ce que** sont prévues deux parties de recouvrement (58), une partie de recouvrement (58) étant appliquée par pivotement respectivement au niveau de chaque extrémité libre de la partie de soutien (54).

3. Dispositif de traitement (10) selon la revendication 1 ou 2, **caractérisé en ce que** la partie de soutien (54) et la partie de recouvrement (58), au moins au nombre de une, se complètent en un cercle.

4. Dispositif de traitement (10) selon l'une des revendications 1 à 3, **caractérisé en ce que** le dispositif de pivotement comporte un élément en forme de plaque (56) au niveau duquel sont prévus un dispositif de rotation permettant une rotation relative entre la partie de soutien (54) et l'élément en forme de plaque (56) et un dispositif de rotation entre l'élément en forme de plaque (36) et la partie de recouvrement (58).

5. Dispositif de traitement (10) selon l'une des revendications précédentes, **caractérisé en ce que** la partie de soutien (54) est prévue sur une surface principale de l'élément en forme de plaque (56) et la partie de recouvrement (58), au moins au nombre de une, sur l'autre surface principale de l'élément en forme de plaque (56).

6. Dispositif de traitement (10) selon l'une des revendications précédentes, **caractérisé en ce que** l'appareil de traitement est un appareil de thérapie par champ magnétique.

7. Dispositif de traitement (10) selon l'une des revendications précédentes, **caractérisé en ce que** le bâti (22) comporte une extrémité de tête inclinable.

8. Dispositif de traitement (10) selon la revendication 7, **caractérisé en ce que** l'extrémité de tête inclinable occupe environ la moitié de la longueur du lit de traitement.

9. Dispositif de traitement (10) selon l'une des revendications précédentes, **caractérisé en ce que** le bâti comporte un corps de support (26) qui s'étend essentiellement horizontalement, une partie de tête (28) applicable par pivotement dans la zone médiane, dans le sens longitudinal, du corps de support, et une partie d'appui (30) pouvant être appliquée par articulation au niveau de la partie de tête, et qui peut s'encliqueter par son extrémité libre dans une zone d'encliquetage prévue au niveau du corps de support (26) pour provoquer une inclinaison de la partie de tête (28).

10. Dispositif de traitement (10) selon la revendication 9, **caractérisé en ce que** sont prévues au niveau du corps de support (26) plusieurs zones d'encliquetage de façon à ce que la partie de tête puisse adopter différentes positions inclinées.

11. Dispositif de traitement (10) selon l'une des revendications précédentes, **caractérisé en ce que** le bâti (22) est en bois.

12. Dispositif de traitement (10) selon l'une des revendications 1 à 10, **caractérisé en ce que** le bâti (22) est en métal.

13. Dispositif de traitement (10) selon l'une des revendications précédentes, **caractérisé en ce que** la surface portante (32, 33), au moins au nombre de une, est une surface portante rembourrée moelleuse.

14. Dispositif de traitement (10) selon l'une des revendications précédentes, **caractérisé en ce que** la surface portante comporte une partie de tête (33) séparée et une partie de pied (32).

15. Dispositif de traitement (10) selon l'une des revendications précédentes, **caractérisé en ce que** la surface portante (32) présente un creusement dans la zone dans laquelle repose le fessier du patient, et est en revanche en relief dans la zone dans laquelle reposent les jambes.

16. Dispositif de traitement (10) selon l'une des revendications précédentes, **caractérisé en ce que** la surface portante (32) présente, dans la zone de l'évidement du bâti, au moins au nombre de un, un évidement (34) qui dégage au moins l'évidement (29) existant dans le bâti, et **en ce qu'**est prévu pour l'évidement dans la surface portante au moins un élément de remplissage (35) pour recouvrir au moins partiellement l'évidement (34).

17. Dispositif de traitement (10) selon l'une des revendications précédentes, **caractérisé en ce que** la surface portante (33) est façonnée dans la zone de l'évidement (34), en creux par rapport à une zone en relief pour soutenir la région des lombaires d'un patient.

18. Dispositif de traitement (10) selon l'une des revendications précédentes, **caractérisé en ce que** le mécanisme de réglage (36) comprend une plaque (37) fixée au niveau du côté opposé au côté de soutien, au niveau de laquelle est appliqué de façon déplaçable le support (38), au moins au nombre de un, pour l'appareil de traitement.

19. Dispositif de traitement (10) selon l'une des revendications précédentes, **caractérisé en ce que** le support (38), au moins au nombre de un, peut être déplacé par une broche et une manivelle (39) permettant d'actionner la broche.

20. Dispositif de traitement (10) selon l'une des revendications précédentes, **caractérisé en ce qu'**est prévu un logement (44) pour l'appareil de génération de signaux et/ou l'appareil de commande (42) d'un appareil de traitement.
